# EUROPÄISCHE PATENTSCHRIFT

(11) **EP 2 768 471 B1**
(45) Veröffentlichungstag und Bekanntmachung des Hinweises auf die Patenterteilung: **20.04.2016**
(21) Anmeldenummer: 12775486.9
(22) Anmeldetag: 17.10.2012
(51) Int. Cl.: A61K 8/02, A61K 8/04, A61K 8/37, A61K 8/63, A61K 8/67, A61K 8/68, A61K 8/73, A61K 8/97, A61Q 19/00, A61K 9/00, A61K 9/10, A61K 9/51, A61K 36/185

(54) **WÄSSRIGE DISPERSION MIT EINEM GEHALT AN LIPIDNANOPARTIKELN, VERFAHREN ZU DEREN HERSTELLUNG SOWIE DEREN VERWENDUNG**
AQUEOUS DISPERSION HAVING A CONTENT OF LIPID NANOPARTICLES, PROCESS FOR PRODUCTION THEREOF AND USE THEREOF
DISPERSION AQUEUSE CONTENANT UNE FRACTION DE NANOPARTICULES LIPIDIQUES, SON PROCÉDÉ DE PRÉPARATION ET SON UTILISATION

(30) Priorität: 18.10.2011 DE 102011116069
(43) Veröffentlichungstag der Anmeldung: 27.08.2014
(73) Patentinhaber: Dr. Rimpler GmbH, 30900 Wedemark (DE)
(72) Erfinder: RIMPLER, Christian, 30900 Wedemark (DE); SCHWABE, Kay, 30900 Wedemark (DE)
(74) Vertreter: Held, Stephan
(86) Internationale Anmeldenummer: PCT/EP2012/070580
(87) Internationale Veröffentlichungsnummer: WO 2013/057144

(56) Entgegenhaltungen:
- WO-A2-2010/051918
- CN-A- 101 822 839
- US-A1- 2006 083 781
- OBEIDAT W M ET AL: "Preservation of nanostructured lipid carriers (NLC)", EUROPEAN JOURNAL OF PHARMACEUTICS AND BIOPHARMACEUTICS, ELSEVIER SCIENCE PUBLISHERS B.V., AMSTERDAM, NL, Bd. 76, Nr. 1, 1. September 2010 (2010-09-01), Seiten 56-67, XP027210083, ISSN: 0939-6411 [gefunden am 2010-08-12]
- LUCIA MONTENEGRO ET AL: "In vitro evaluation of idebenone-loaded solid lipid nanoparticles for drug delivery to the brain", DRUG DEVELOPMENT AND INDUSTRIAL PHARMACY, NEW YORK, NY, US, Bd. 37, Nr. 6, 1. Juni 2011 (2011-06-01), Seiten 737-746, XP009172273, ISSN: 0363-9045, DOI: 10.3109/03639045.2010.539231
- CASTRO G A ET AL: "Development of a new solid lipid nanoparticle formulation containing retinoic acid for topical treatment of acne", JOURNAL OF MICROENCAPSULATION, TAYLOR AND FRANCIS, BASINGSTOKE, GB, Bd. 24, Nr. 5, 1. August 2007 (2007-08-01) , Seiten 395-407, XP002696370, ISSN: 0265-2048, DOI: 10.1080/02652040701288519
- KHALIL MITRI ET AL: "Lipid nanocarriers for dermal delivery of lutein: Preparation, characterization, stability and performance", INTERNATIONAL JOURNAL OF PHARMACEUTICS, ELSEVIER BV, NL, Bd. 414, Nr. 1, 2. Mai 2011 (2011-05-02), Seiten 267-275, XP028233951, ISSN: 0378-5173, DOI: 10.1016/J.IJPHARM.2011.05.008 [gefunden am 2011-05-07]

## Beschreibung

### Technisches Gebiet

Die vorliegende Erfindung betrifft eine wässrige Dispersion zur kosmetischen und pharmazeutischen Applikation mit einem Gehalt an darin dispergierten, negativ oder positiv geladenen Primärteilchen einer Teilchengröße von 10 bis 1000 nm, wobei die Primärteilchen eine Lipidmatrix auf Basis von bei 20 °C festen Lipiden aufweisen und Wirkstoffe enthalten, ein Verfahren zu deren Herstellung und deren Verwendung für die kosmetische und pharmazeutische Applikation von Wirkstoffen, insbesondere auf die Haut.

### Stand der Technik

Nanodispergierte Systeme, wie Liposome, Nanoemulsionen und Lipid Nanopartikel, haben in den letzten Jahren als potentielle Vehikel für die kontrollierte Applikation von Kosmetika und für die optimierte Abgabe von Wirkstoffen an bestimmte Hautschichten Bedeutung erlangt. Solid Lipid Nanopartikel stellen dabei ein alternatives Trägersystem zu traditionellen kolloidalen Trägersystemen, wie Emulsionen, Liposomen und polymerbasierten Micro- oder Nanopartikeln, dar.

Solid Lipid Nanoparticles (SLNs) wurden zu Beginn der 1990er Jahre entwickelt und entstehen durch das Ersetzen eines flüssigen Öls in einer Öl-in-Wasser-Emulsion durch ein festes Lipid oder ein Gemisch fester Lipide. Die festen Lipide sind sowohl bei Raumtemperatur als auch bei Körpertemperatur fest. SLNs bestehen in der Regel aus 0,1 - 30% (w/w) des festen Lipids, das in wässrigem Medium durch 0,5 bis 5% (w/w) Emulgator stabilisiert wird. Der durchschnittliche Partikeldurchmesser von SLNs liegt im Bereich von 40 bis 1000 nm.

Eine Weiterentwicklung von festen Lipid Nanopartikeln stellen die sogenannten nanostrukturierten Lipidträger (NLCs) dar, die aus Mischungen von festen Lipiden und flüssigen Lipiden (Ölen) in einem Verhältnis von 70:30 bis 99,9:0,1 bestehen. Nano-strukturierte Lipidträger weisen aufgrund dieser Lipidmischungen im Vergleich zu den auf ausschließlich festen Lipiden basierenden Nanopartikeln einen geringeren Schmelzpunkt auf, sind jedoch genau wie diese bei Körpertemperatur (37°C) fest. Im Vergleich zu SLNs weisen NLCs für eine Reihe von Wirkstoffen eine höhere Beladungskapazität auf, erlauben die Formulierung von Partikelsuspensionen mit geringerem Wassergehalt und zeigen eine verminderte Abgabe des Wirkstoffs während der Lagerung.

Bei der Erzeugung von SLNs oder NLCs ist gemäß dem Stand der Technik der Einsatz von Emulgatoren, wie Sojabohnenlecithin, Eilecithinphosphatidylcholin, Polaxomer, Natriumcholate/Glycolat/Butanol, Butyrsäure oder Taurocholsäure Natriumsalz, erforderlich

Für die Herstellung von Solid Lipid Nano Particles können verschiedene Verfahren angewandt werden, wie beispielsweise die Hochdruckhomogenisierung, die Mikroemulsionstechnik, die Emulgierung mit anschließender Lösungsmittelverdampfung, die Emulgierung mit anschließender Abdiffusion des Lösungsmittel, die Lösungsmittelinjektionsmethode oder die Phaseninversionsemulgierung. Von diesen weist die Hochdruckhomogenisierung verschiedene Vorteile gegenüber den anderen Verfahren auf. Beispielsweise lässt sich die Ansatzgröße leicht variieren und der Einsatz von organischen Lösungsmitteln kann vermieden werden. Darüber hinaus ist die Produktionszeit gering.

Bei der Hochdruckhomogenisierung können zwei Verfahren unterschieden werden, die als kalte Hochdruckhomogenisierung oder heiße Hochdruckhomogenisierung bezeichnet werden. Bei der kalten Hochdruckhomogenisierung wird der Wirkstoff zunächst in eine Schmelze des festen Lipids eingemischt, die abkühlt und im festen Zustand zu Lipid-Micropartikeln vermahlen wird. Diese werden in einer kalten Emulgatorlösung suspendiert und im Hochdruckhomogenisator zu Nanopartikeln verarbeitet. Bei der heißen Hochdruckhomogenisierung wird der Wirkstoff ebenfalls zunächst in einer Schmelze des festen Lipids dispergiert und anschließend in einer heißen Emulgatorlösung dispergiert. Die erhaltene Dispersion wird dann ebenfalls im Hochdruckhomogenisator in eine Nanopartikel-Dispersion umgewandelt, in der sich die Nanopartikel beim anschließenden Abkühlen verfestigen.

Beispielhaft für im Stand der Technik beschriebene SLNs sowie Verfahren zu deren Herstellung sei auf die folgenden Druckschriften verwiesen: In den WO 2002/051390 A1, WO 2001/003652 A1 und DE 4131562 A1 werden mit unterschiedlichen Wirkstoffen beladene Solid Lipid Nanoparticles (SLNs) beschrieben, die durch den Prozess der Hochdruckhomogenisierung gewonnen werden. Die in diesen Schriften beschriebenen Nanopartikel enthalten als Emulgatoren wirksame amphiphile Substanzen, wie u.a. Sphingolipide (Ceramide) und Sterine (z.B. das Phytosterol Stigmaterin). In der KR 2007 038 606 und KR 2007 074 223 werden Nanokapseln beschrieben, die Ceramide enthalten. Zusätzlich werden als Emulgatoren Alkohole mit mehreren OH-Gruppen eingesetzt. Die Herstellung dieser Nanokapseln soll ohne Verwendung eines Hochdruckhomogenisators möglich sein. In der WO 2008/133482 A1 werden Ceramidnanokapseln für kosmetische Anwendungen beschrieben. Diese Nanokapseln weisen eine Polymerhülle auf, die zu ihrer Stabilisierung beiträgt.

Die US 2011/0201695 A1 offenbart in Öl-in-Wasser-Emulsionen eingekapselte therapeutische Wirkstoffe auf Basis von festen Lipiden und Amphiphilen, die über eine Präemulsion und die anschließende Homogenisierung dieser Präemulsion hergestellt werden. Diese Nanoemulsionen sind für u.a. topische Anwendungen vorgesehen und weisen ein Zetapotential von -20 und 20 mV auf. Ein solches Zetapotential ist jedoch ungünstig, da Dispersionen mit einem Zetapotential im Bereich von -30 bis 30 mV auf instabile Systeme hindeuten, die zur Flockung neigen.

Die EP 1 606 044 B2 beschreibt eine Vorrichtung und ein Verfahren zur kontinuierlichen Herstellung von Emulsionen oder Dispersionen, speziell zur Herstellung von Nanoemulsionen, die pharmazeutische, kosmetische und/oder lebensmitteltechnologische Wirkstoffe enthalten und auf Lipiden beruhen. Diese Emulsionen stellen SLNs- und Nanodispersionen dar, die durch klassisches Emulgieren der Gemische bei hohen Drehzahlen erhalten werden. Dieses Dokument beschreibt kein Zetapotential für die erhaltenen Produkte, so dass unklar ist, ob es sich bei diesen um langfristig stabile Dispersionen handelt.

Die US 2009/0238878 offenbart pharmazeutische Zusammensetzungen, die auf festen Nanopartikeln, die in einem im Wesentlichen aus Wasser bestehen Medium dispergiert sind, beruhen. Die Nanopartikel enthalten unlösliche pharmazeutische Wirkstoffe wie Docetaxel und weisen eine reduzierte Ostwald-Reifung auf. Als Bestandteile der Nanopartikel beschreibt dieses Dokument u.a. Fettsäuren, die in Gegenwart von Emulgatoren zu Nanopartikeln umgesetzt und anschließend hochdruckhomogenisiert werden. Auch diese Druckschrift beschreibt jedoch eine klassische Emulgation zur Herstellung der Nanopartikel.

Die US 2006/083781 A1 beschreibt schließlich SLNs mit neutralen Lipiden und funktionalisierten Polymeren, insbesondere amphiphilen Polymeren, die eine nach außen ragende polare Gruppe tragen, sowie deren Herstellung in Dispersion. In die entstehenden Partikel werden magnetische Materialien eingebracht und deren potentielle Verwendung als Quantenpunkte untersucht. Die hergestellten Partikel weisen ein Zetapotential (abhängig vom pH-Wert) von 10 bis -20 mV auf.

Weiterhin sind in G. Deli et al., (Journal of Liposome Research, 2009, 19(3), S. 180-188) mit Ceramiden beladene Nanoemulsionen und Solid Liquid Nanoparticles (SLNs) beschrieben. Für die Herstellung dieser Partikel werden Emulgatoren, wie Sojaphosphatidylcholin und Solutol, eingesetzt, wobei die Partikel über ein Doppel-Emulsionsverfahren erzeugt wurden. Van Lummel et al. beschreibt in FASEB Journal (2011, 25(1), S. 280-289) mit kurzkettigen Ceramiden angereicherte Lipidnanovisikel zur verbesserten Darreichung des Wirkstoffs Doxorubicin. Schließlich findet sich im Review von Puri et al., Journal of Globular Pharmatechnology, 2010; 2(9), S. 1-15, ein Überblick über den derzeitigen Stand der Technologie bei Lipidnanopartikeln.

Obeidat et al. "Preservation of nanostructured lipid carriers (NLC)", European Journal of Pharmaceutics and Biopharmaceutics, Elsevier Science Publishers B.V., Amsterdam, NL, Bd. 76, Nr. 1, 1. September 2010, S. 56-67 befasst sich mit der Stabilisierung von NLC-Dispersionen, wobei diese anhand von Emulgatoren erfolgt. Untersucht wird darüber hinaus der Einfluss von Konservierungsmitteln auf die physikalische Stabilität von NLC-Dispersionen. Es wurde festgestellt, dass Konservierungsmittel keinen Einfluss auf die Stabilität der NLC haben.

Bei den in klassischen SLNs verwendeten Emulgatoren handelt es sich überwiegend um nicht körpereigene Stoffe, die bei Applikation insbesondere auf empfindlichen oder schon vorgeschädigten Hautstellen zu weiteren Irritationen führen können.

Trotz der beschriebenen Fortschritte in der Technologie besteht ein Bedarf an Nanopartikeln, die keine nicht natürlichen Emulgatoren enthalten und deren Zusammensetzung im Wesentlichen der Zusammensetzung an ihrem Applikationsort, beispielsweise der Haut, entspricht. Insbesondere besteht ein Bedarf nach einem Trägersystem, dass den natürlichen Gegebenheiten der äußersten Hautschicht (dem Stratum Corneum) und seiner Lipide angepasst ist. Dabei soll die wünschenswerte Stabilität von mit nicht natürlichen Emulgatoren hergestellten SLNs während der Lagerung erhalten bleiben.

Ein weiterer Nachteil von SLNs, bei denen der Wirkstoff in die die Lipidmatrix dispergiert wurde, besteht darin, dass die SLNs bei Körpertemperatur fest sind. Somit kann der Wirkstoff nur über einen längeren Zeitraum an den Applikationsort abgegeben werden, der sich nach der Abbauzeit der Partikel bemisst. Es besteht daher ein Bedarf an SLNs, bei denen der Wirkstoff nur in den äußeren Bereich der Partikel eingearbeitet wird, so dass die Partikel an der Oberfläche eine hohe Wirkstoffkonzentration aufweisen, aber in ihrem Inneren nahezu wirkstofffrei sind.

### Gegenstand der Erfindung

Gelöst werden diese Probleme erfindungsgemäß durch eine wässrige Dispersion zur kosmetischen Applikation mit einem Gehalt an darin dispergierten, negativ oder positiv geladenen Primärteilchen einer Teilchengröße von 10 bis 1000 nm, wobei die Primärteilchen eine Lipidmatrix auf Basis von bei 20 °C festen Lipiden aufweisen und Wirkstoffe sowie gegebenenfalls weitere Additive enthalten, die dadurch gekennzeichnet ist, dass die Wirkstoffe amphiphil und in der Grenzfläche der Primärteilchen so verankert sind, dass deren lipophiler Anteil nach innen in die Primärteilchen und deren hydrophiler Anteil nach außen in die Dispersion weist, wobei die amphiphilen Wirkstoffe als Ceramide, Phytosterole, Lipoaminosäuren oder Lipoproteine vorliegen. Nachfolgend wird im Zusammenhang mit den oben angesprochenen Primärteilchen einer Teilchengröße von 10 bis 1000 nm von "nanoskaligen Primärteilchen" gesprochen.

Bei kosmetischen Verwendungen der erfindungsgemäßen wässrigen Dispersion ist es bevorzugt, wenn die nanoskaligen Primärteilchen eine Teilchengröße von 150 bis 220 nm aufweisen.

Ein weiterer Aspekt der vorliegenden Erfindung betrifft ein Verfahren zur Herstellung solcher wässriger Dispersionen, wobei das Verfahren dadurch gekennzeichnet ist, dass eine Präemulsion der Ausgangsmaterialien nach der PIT-Technologie (Phaseninversionstemperatur/Phasenvolumenfeld) hergestellt, die Präemulsion einer Hochdruckhomogenisation unterzogen und anschließend die erhaltene wässrige Dispersion der nanoskaligen Primärteilchen abgekühlt wird.

Schließlich betrifft die vorliegende Erfindung die Verwendung der vorstehenden beschriebenen Dispersion zur kosmetischen und pharmazeutischen Applikation von Wirkstoffen, insbesondere auf der Haut, insbesondere auf Barriere-geschädigter Haut.

In der vorstehend beschriebenen wässrigen Dispersion liegt der Anteil an nanoskaligen Primärteilchen vorzugsweise im Bereich von 5 bis 70 Gew.-%, insbesondere im Bereich von 5 bis 45 Gew.-%. Weiterhin ist die wässrige Dispersion dann vorteilhaft ausgestaltet, wenn ihr Zetapotential (bestimmt nach der Messmethode in Abhängigkeit vom pH-Wert mit dem Gerät Zetasizernano ZS, Malvern Instruments) weniger als 200 mV, insbesondere weniger als 100 mV, jedoch mehr als 0 mV, insbesondere mehr als 30 mV, beträgt. Demzufolge liegt das Zetapotential bevorzugt zwischen 200 mV und 30 mV. Besonders bevorzugt liegt das Zetapotential im Bereich von 80 mV bis 40 mV, insbesondere ist es kleiner als 70 mV. Am meisten bevorzugt ist es, wenn das Zetapotential im Bereich von 50 bis 68 mV liegt. Die obigen Angaben des Zetapotentials haben entweder ein Minus-Vorzeichen oder ein Plus-Vorzeichen. Entscheidend ist es, dass sie eine hinlängliche Ladung, ob negativ oder positiv, aufweisen, damit sie die wünschenswerte Stabilisierung der erfindungsgemäßen Dispersion bewirken.

Hinsichtlich der als Lipidmatrix zu verwendenden Lipide unterliegt die wässrige Dispersion gemäß der vorliegenden Erfindung keinen relevanten Beschränkungen. Es ist allein erforderlich, dass die Lipide der Lipidmatrix bei Raumtemperatur (20 °C) in fester Form vorliegen. Bevorzugt sind die festen Lipide ausgewählt aus der Gruppe bestehend aus Fettalkoholen, insbesondere Cethylalkohol, Fettsäureestern, insbesondere Partialfettsäureestern, insbesondere in Form von Cetylpalmitat, Isopropylpermitat, oder Octylpalmitat, Fettsäuren, Steroiden, Terpenen, Saponinen, Wachsen, natürlich und/oder derivatisiert, insbesondere in Form von Bienenwachs und Canaubawachs, veresterten Proteinen, insbesondere Cerumiden (I und II) und/oder Phytosterole, dies sowohl natürlich als auch derivatisiert.

Die Menge der Lipide, bezogen auf die wässrige Dispersion, liegt in der Regel im Bereich von 5 bis 40 Gew.-%, bevorzugt im Bereich von 10 bis 35 Gew.-%, und insbesondere im Bereich zwischen 10 und 25 Gew.-%. Abzüglich des Wasseranteils liegt der Anteil des Lipids oder der Lipide an den nanoskaligen Primärteilchen vorzugsweise im Bereich von 6 bis 85% und insbesondere zwischen 55 und 70 Gew.-%.

Die Stabilisierung der erfindungsgemäßen Dispersion, die die bezeichneten nanoskaligen Primärteilchen enthält, beruht auf der Verwendung von amphiphilen Substanzen und gegebenenfalls der zusätzlichen Verwendung eines Polysaccharidderivats zur sterischen Stabilisierung. Bei diesen amphiphilen Wirkstoffen handelt es sich um solche in Form von Ceramiden, insbesondere Aminopropandiolestern, Phytosterolen, insbesondere des Granatapfels oder der Acaibeere, Lipoaminosäuren, insbesondere Natriumcocoylalaninat oder Palmitoylglycin, oder Lipoproteine.

Diese Inhaltsstoffe verbessern, neben ihrer besonderen Art die Haut zu schützen, bei in einer Emulsionsmatrix eingebetteten Lipidnanopartikeln auch das thixotrope Verhalten der Emulsion. So konnte gezeigt werden, dass Ö-in-Wasser (OW)-Emulsionen mit Fettalkoholen innerhalb einer Matrix interagieren. Die Fließgrenze eines Fertigprodukts von beispielsweise kosmetischen OW-Emulsionen mit Lipidnanopartikeln erhöhte sich, während das thixotrope Verhalten wesentlich stärker ausgeprägt war. Phytosterole aus dem Granatapfel oder der Acaibeere Tocopherole, Lipoaminosäuren, Retinol, und Ascorbylpalmitat sind zudem besonders gut verträglich und einfach verarbeitbar und verbessern dadurch die Produktleistung.

Mit besonderen Vorteilen ist es verbunden, wenn die amphiphilen Wirkstoffe in Form von Phytosterolen aus dem Granatapfel (Punica Granatum Sterolis), Euterpe Oleracea Sterolis (Phytosterole aus der Acaibeere) Leinsamenöl, Palmöl, Aminopropandiolester (synthetisches Ceramid), Sonnenblumenöl, Palmöl-Aminopropanolester, Natrium Cocoyl Alaninat (Lipoaminosäure) vorliegen.

Hinsichtlich der Menge der einzusetzenden amphiphilen Wirkstoffe unterliegt die vorliegende Erfindung keinen relevanten Beschränkungen. Es hat sich als zweckmäßig erwiesen, wenn die Menge der amphiphilen Wirkstoffe, bezogen auf die Primärteilchen zwischen 1 bis 60 Gew.-%, insbesondere zwischen 10 und 45 Gew.-% und besonders bevorzugt zwischen 15 und 40 Gew.-% beträgt.

Die Freisetzung von amphiphilen Wirkstoffen aus den erfindungsgemäß eingesetzten nanoskaligen Primärteilchen ist bei topischer Applikation wesentlich erleichtert, wenn die Wirkstoffe in eine Lipidmatrix integriert sind, die dem Stratum Corneum Lipide sehr ähnlich ist. Die Wirkstofffreisetzung erfolgt in solchen nanoskaneum Lipide sehr ähnlich ist. Die Wirkstofffreisetzung erfolgt in solchen nanoskaligen Primärteilchen nicht überwiegend auf der Hautoberfläche, sondern unmittelbar innerhalb der lamellaren Doppelschicht des Stratum Corneum. Somit ist eine Verwendung nicht nur als kosmetischer Wirkstoff und Emulsions-performanceverbesserer, sondern auch als topisches Wirkstoffträgersystem für Pharmazeutika geeignet.

Im Rahmen der vorliegenden Erfindung ist es daher bevorzugt, wenn die Bestandteile der erfindungsgemäß eingesetzten nanoskaligen Primärteilchen überwiegend aus hauteigenen Bausteinen, insbesondere Ceramiden, Cholesterolen und freien Fettsäuren, bestehen. Diese Bestandteile sind beispielsweise als pflanzliche oder halbsynthetische Rohstoffe pflanzlicher Herkunft erhältlich. Im Zusammenhang mit der vorliegenden Erfindung ist es hingegen nicht bevorzugt, wenn in der Zusammensetzung synthetische Rohstoffe nicht natürlichen Charakters (d.h. die nicht in natürlichen Systemen vorkommen und nicht verstoffwechselbar sind) enthalten sind.

Zusätzlich zu Lipiden und amphiphilen Wirkstoffen kann die wässrige Dispersion anwendungsspezifische Wirkstoffe für die Haut enthalten, die in die nanoskaligen Primärteilchen integriert sind. Bei solchen anwendungsspezifischen Wirkstoffen handelt es sich insbesondere um Phytosterole aus dem Granatapfel, Phytosterole aus der Accaibeere, Lipoaminosäuren, Peptidestern, synthetische Ceramide, Ceramide I und II, lipophile/lipophilisierte Vitamine, insbesondere Retinol, Tocopheron, Ubichinon und Ascorbyl-Palmitat.

Darüber hinaus kann es von Vorteil sein, wenn die nanoskaligen Primärteilchen zusätzliche Additive in Form von Antioxidationsmitteln, insbesondere Tocopherole, Ascorbyl-Palmitat, Retinol und Derivate hiervon, acylierte Polysaccharide, Feuchthaltemittel, wie Pentylenglycol oder Caprilylglycol, und/oder Konservierungsmittel, insbesondere in Form von Phenoxyethanol oder Etylhexylglycerin, enthalten. Es hat sich als zweckmäßig erwiesen wenn die Lipidprimärpartikel einen Gehalt an solchen zusätzlichen Inhaltsstoffen von bis zu etwa 5 Gew.-%, insbesondere von bis zu etwa 1 Gew.-% bezogen auf die Primärpartikel, aufweisen.

Zur Unterstützung und Stabilisierung können die nanoskaligen Primärteilchen durch angelagerte Stabilisatoren weiter modifiziert werden, um das Agglomerieren der einzelnen Partikel im Dispersionsmedium nach der Fertigung zu verhineine sterische Hinderung bewirken, insbesondere in Form von acylierten Polysacchariden und crosspolymerisierten, acylierten Acrylaten. Bei den als Stabilisator verwendeten acylierten Polysacchariden handelt es sich vorzugsweise um solche auf Inulinbasis, insbesondere Inulinlaurylcarbamat, oder solche auf Guarbasis, insbesondere in Form von C₁₈-C₂₂ Hydroxyalkylhydroxydpropylguar, die eine lipophile Seitenkette sowie ein hydrophiles Zuckergerüst aufweisen. Diese Substanzen stabilisieren die nanoskaligen Primärteilchen, in dem der lipophile Seitenkettenrest in das Primärteilchen hineinragt, während die hydrophile Saccharidkette eine Hülle um die Nanoteilchen bildet, die mit dem umgebenden Wasser in Kontakt tritt. Durch die hydrophile Hülle wird der Zerfall sowie ein Agglomerieren der einzelnen nanoskaligen Primärteilchen unterbunden. Die Menge des Stabilisators in der Gesamtmischung ist nicht wesentlich. Es hat sich als zweckmäßig erwiesen, wenn der Stabilisator in einer Menge von weniger als etwa 30 Gew.-%, insbesondere von etwa 17 bis 25 Gew.-%, bezogen auf das Gesamtgewicht der nanoskaligen Primärteilchen, vorliegt.

Wie vorstehend ausgeführt, betrifft die vorliegende Erfindung auch ein Verfahren zur Herstellung einer wässrigen Dispersion, bei dem zunächst eine Präemulsion aus den Ausgangsmaterialien mit Hilfe der PIT Technologie hergestellt wird.

Gegenstand der Erfindung ist demzufolge auch ein Verfahren zur Herstellung einer wässrigen Dispersion gemäß der Erfindung, das dadurch gekennzeichnet ist, dass eine Präemulsion der Ausgangsmaterialen nach der PIT-Technologie (Phasen-Inversions-Temperatur/ Phasen-Volumen-Verhältnis) hergestellt, die Präemulsion einer Hochdruckhomogenisation unterzogen und anschließend die erhaltene wässrige Dispersion der nanoskaligen Primärteilchen abgekühlt wird.

Es wurde überraschenderweise festgestellt, das sich mit dieser Technologie Partikel herstellen lassen, die, wenn sie amphiphile Wirkstoffe enthalten, einen hohen Anteil dieser Wirkstoffe an der Grenzfläche der Primärteilchen aufweisen, wobei der hydrophile Anteil nach außen in die Dispersion weist. In den nach diesem Verfahren hergestellten Partikeln ist in Gegensatz zum Stand der Technik nur ein geringer Anteil des Wirkstoffs im Kern der Partikel enthalten. Dies hat den Vorteil, dass der Wirkstoff deutlich schneller freigesetzt werden kann als bei klassischen SLNs, da in diesen zunächst die feste Lipidmatrix aufgelöst werden muss, bevor der Wirkstoff aus dem Inneren der Partikel freigesetzt wird. In einer bevorzugten Ausführungsform umfasst die vorliegende Erfindung daher auch wässrige Dispersionen, wie sie vorstehend beschrieben sind, die mit Hilfe der PIT-Technologie herstellbar sind.

Das vorstehend beschriebene Verfahren zur Herstellung von wässrigen Dispersionen ist vorzugsweise dadurch weitergebildet, dass die Hochdruckhomogenisation mittels einer Gegenstrahlhochdruckhomogenisation durchgeführt wird. Bei der Hochdruckhomogenisation beträgt der Druck zweckmäßigerweise 400 bis 1300 bar, insbesondere 800 bis 1200 bar.

Weiterhin kann das Verfahren dadurch vorteilhaft ausgestaltet werden, indem bei der Hochdruckhomogenisation ein Temperaturbereich von 30 bis 90°C, insbesondere von 40 bis 60°C, eingestellt wird. Das abschließende Abkühlen der nanoskaligen Primärteilchen wässriger Dispersion kann insbesondere mittels einer Schockkühlung durchgeführt werden.

Die vorstehend beschriebene Verwendung der Dispersion zur kosmetischen und pharmazeutischen Applikation von Wirkstoffen, insbesondere auf der Haut, ist mit besonderen Vorteilen verbunden, wenn in den nanoskaligen Primärteilchen ein immobilisierter weiterer Wirkstoff vorliegt. Bei einem solchen Wirkstoff kann es sich beispielsweise um eine Verbindung zur Behandlung von Neurodermitis handeln, die mit Hilfe der Dispersion direkt auf die Haut appliziert wird. Insbesondere kann es sich bei dem Wirkstoff um Silber-Nanopartikel handeln. Der Wirkstoff kann vollständig in den Primärpartikeln immobilisiert sein, oder er kann nur zu einem Teil in den Partikeln immobilisiert sein, wobei der Rest des Wirkstoffs in der Dispersion vorliegt. Die vorstehend beschriebenen Primärpartikel ermöglichen die Immobilisierung spezieller Wirkstoffe, die mit Techniken gemäß dem Stand der Technik in derartigen Partikeln nicht immobilisierbar sind. Dabei kann das Zeta-Potential der Dispersion dergestalt gesteuert werden, dass auf den jeweiligen Wirkstoff abgestimmt eine besonders vorteilhafte Immobilisierung erzielt werden kann.

Darüber hinaus kann auch ein im lebensmittelchemischen Bereich einsetzbarer Wirkstoff in die nanoskaligen Primärteilchen der Lipidmatrix eingearbeitet werden, beispielsweise ein Vitamin, und insbesondere Vitamin E oder ein Derivat davon. Demzufolge umfasst die vorliegende Erfindung auch die Verwendung entsprechend modifizierter Dispersionen als Lebensmittelzusatzstoff.

Die vorstehend beschriebenen wässrigen Dispersionen mit den darin dispergierten nanoskaligen Primärteilchen weisen vielfältige Vorteile auf:
Die Zusammensetzung der nanoskaligen Primärteilchen kann so gewählt werden, dass sie mit ihrem Applikationsort nahezu identisch ist. Phytostyrole ähneln als feste Lipidkomponenten den humanen Cholesterolverbindungen, welche für den Aufbau der lamellaren Doppelschicht im Stratum Corneum Lipide von zentraler Bedeutung sind. Ähnliches gilt für halbsynthetische Ceramide als feste Lipidkomponente, da Ceramide und ungesättigte Fettsäuren eine weitere Komponente des Stratum Corneum Lipides darstellen. Die Integrität des Stratum Corneum Lipidae ist für eine Gesunderhaltung der Haut und des Körpers von großer Bedeutung.

Der monopartikuläre Lipidfilm der Lipidpartikel verstärkt durch seine Zusammensetzung den hauteigenen Hydrolipidfilm, der als äußerste Barriere gegen externe Reizstoffe fungiert und darüber hinaus den transepidermalen Wasserverlust reguliert. Dieser Wirkmechanismus stellt einen wesentlichen Vorteil bei der topischen Anwendung von erfindungsgemäßen nanoskaligen Primärteilchen dar. Fehlende Bereiche des Hydrolipidfilms auf der Hautoberfläche lassen sich durch die Ausbildung eines monopartikulären Films auffüllen und sichern so die Integrität des äußeren Schutzes.

Darüber hinaus erlaubt eine solche Zusammensetzung der nanoskaligen Primärteilchen nicht nur eine Verwendung als Trägersystem, sondern die Primärteilchen können auch selbst als Lieferant für im Stratum Corneum benötigte Bausteine dienen und als Wirkstoff eingesetzt werden, der äußerst verträglich und nicht biopersistent ist. Beispielsweise können die Lipidnanopartikel ausgewaschene und beschädigte Bereiche des Stratum Corneum Lipide mit Bausteinen versorgen, die durch topische Applikation aufgebracht werden können, und so ähnlich einem Reparaturmörtel die Reparatur der beschädigten Stellen beschleunigen.

Insbesondere sind die erfindungsgemäßen nanoskaligen Primärteilchen in der Lage, einen hauteigenen Hydrolipidfilm schnell zu schließen und helfen, ihn langfristig mit Ceramiden und Phytosterolen wieder aufzubauen. Aus diesem Grunde eignen sich die erfindungsgemäßen Dispersionen insbesondere zur Pflege von schwer vorgeschädigter bzw. Barriere-gestörter Haut. Der semipermeable Schutzfilm hält neben chemischen Noxen auch mikrobielle Belastungen von der geschädigten Haut fern und unterstützt so den natürlichen Heilungsprozess, insbesondere in Therapiepausen. Dies führt dazu, dass beispielsweise bei atopischer Dermatitis auf eine Behandlung mit Cortison oder Hydrocortison verzichtet werden kann.

Neben der Verwendung als Wirkstoffsystem in topischen Formulierungen ist die Implementierung von zusätzlichen lipophilen und lipophilisierten Wirkstoffen innerhalb der Lipidmatrix möglich, die in dieser Formulierung von äußeren Einflüssen geschützt bleiben.

Die Teilchengröße der nanoskaligen Primärteilchen bzw. Lipidnanopartikel ist mittels der Hochdruckhomogenisation im Gegenstrahlverfahren steuer- und kontrollierbar. Durch die Art und Zusammensetzung der Partikelmatrix wird daher ein System zur Verfügung gestellt, das die topische Wirkstoffapplikation nicht nur für kosmetische Anwendungen optimiert, sondern auch für pharmazeutische topische Applikationen den Wirkstoff/Medikamententransport bei topischer Applikation optimiert und verbessert.

Schließlich sind die erfindungsgemäßen wässrigen Dispersionen aufgrund der festen Struktur der nanoskaligen Primärteilchen innerhalb des Emulsionssystems stabil. Die Primärteilchen können daher in einfacher Weise durch Einarbeiten der Wirkstoffe in OW (Öl-in-Wasser)-Formulierungen eingearbeitet werden und sorgen bei topischen Applikationen für eine Performanceverbesserung bestehender Formulierungen.

Im Folgenden wird die vorstehend beschriebene Erfindung im Rahmen illustrierender Beispiele eingehend dargelegt.

### Beispiele

Es werden verschiedene wässrige Dispersionen von nanoskaligen Primärteilchen, basierend auf den folgenden Zusammensetzungen hergestellt.

### Zusammensetzung 1 (Ceraparts^{®} Ω6)

| **Rohstoff** | **INCI** | **%** |
|---|---|---|
| Demineralisiertes Wasser | AQUA | 65,9 |
| Cutina CP | Cetyl Palmitate | 21 |
| ABS Pommegranate Sterole | Punica Granatum Sterols | 7 |
| Inutec SP1 | Inulin Lauryl Carbamate | 4 |
| Omega 6 Ceramide | Sunflower Oil / Palm Oil Aminopropandiolesters | 2 |
| Controx KS | Tocopherol | 0,08 |
| | Hydrogenated Palm Glycerides Citrate | 0,02 |

### Zusammensetzung 2 (Ceraparts^{®} AA)

| **Rohstoff** | **INCI** | **%** |
|---|---|---|
| Demineralisiertes Wasser | AQUA | 69,9 |
| Cutina CP | Cetyl Palmitate | 19 |
| Inutec SP1 | Inulin Lauryl Carbamate | 5 |
| Survicode | Natrium Cocoyl Alaninate | 3 |
| Omega 6 Ceramide | Sunflower Oil/Palm Oil Aminopropandiolesters | 2 |
| ABS Pommegranate Sterole | Punica Granatum Sterols | 1 |
| Controx KS | Tocopherol | 0,08 |
| | Hydrogenated Palm Glycerides Citrate | 0,02 |

### Zusammensetzung 3 (Ceraparts^{®} Ω3)

| **Rohstoff** | **INCI** | **%** |
|---|---|---|
| Demineralisiertes Wasser | AQUA | 65,9 |
| Cutina CP | Cetyl Palmitate | 21 |
| ABS Pommegranate Sterole | Punica Granatum Sterols | 7 |
| Inutec SP1 | Inulin Lauryl Carbamate | 4 |
| Omega 3 Ceramide | Palm Oil Aminopropandiolesters | 1 |
| | Linseed oil | 1 |
| Controx KS | Tocopherol | 0,08 |
| | Hydrogenated Palm Glycerides Citrate | 0,02 |

In den Tabellen bezeichnet INCI die "International Nomenclature of Cosmetic Ingredients".

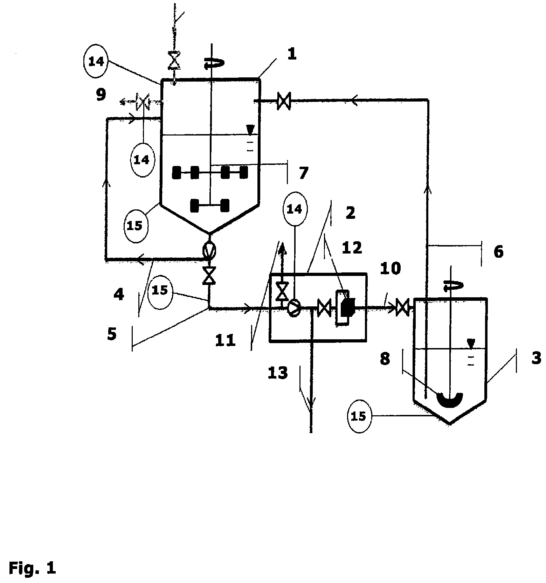

Nachfolgend wird auf die Figur 1 (Anlage zur Herstellung von erfindungsgemäßen Lipidnanopartikeldispersionen) Bezug genommen, in der 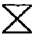 eine Armatur (ein Ventil oder eine Klappe) symbolisiert, während 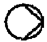 für eine Pumpe, bzw. Kolbenpumpe steht.

Für die Herstellung von Dispersionen mit nanoskaligen Primärteilchen wurden zunächst die lipophilen und amphiphilen Komponenten in einer Vakuummischanlage (1) (Ekatosystems SRC 250) vorgelegt (Phase I). Den lipophilen/amphiphilen Komponenten wurde der sterische Stabilisator hinzugefügt. Die Wasserphase (bestehend in den vorliegenden Beispiel ausschließlich aus Wasser) wurde in einem separaten Vorphasenbehälter (3) mit einem Propellerrührwerk (8) vorgelegt (Phase II). Anschließend wurden beide Phasen getrennt auf 80°C erhitzt. Nach dem Erreichen dieser Temperatur wurde die wässrige Phase langsam unter starkem Rühren mit Hilfe eines Kammerschaufelrührwerks (7) über die Förderleitung (6) in die Lipidphase eingearbeitet, wobei bei einem bestimmten Phasenvolumenverhältnis eine Phaseninversion erfolgte. Der Transport der Wasserphase (3) zu der Lipidphase (1) erfolgte durch ein auf der Vakuummischanlage (1) angelegtes Vakuum (9) Die grobe Emulsion wurde dann bei 4000 UpM mit einem Rotor-Statorsystem (symbolisiert durch das Pumpensymbol am Boden der Anlage (1) im Umlauf (4) homogenisiert, wobei die beabsichtigte Tröpfchengröße der heißen Pränanoemulsion im Bereich von 1 bis 5 µm lag.

Anschließend wurde die Pränanoemulsion mittels Beaufschlagung der Anlage (1) mit Luftdruck über einen Sterilfilter durch Öffnen des untersten Bodenventils der Anlage (1) bei einem Überdruck von etwa 1,5 bar über die beheizbare Produktleitung (5) zur Hochdruckhomogenisieranlage (2) überführt. Der Produktstrom wurde zuerst über die Bypassleitung geführt, um die mit kleinem Querschnitt ausgestatteten Produktleitung innerhalb der Anlage (2) zusätzlich vorzuwärmen, damit es zu keiner Auskristallisation der Lipidmatrix unmittelbar in der Gegenstrahlkammer kommt. Nach ausreichender Vortemperierung wurde die heiße Pränanoemulsion über die Gegenstrahlkammer/Turbulenzkammer (12) geführt (Düsendurchmesser Düsen ca. 0,08 - 0,25 mm). Das Zerkleinerungsprinzip der Tröpfchen der Pränanoemulsion beim Eintritt in die Turbulenzkammer beruht dabei auf Kavitation, Prallkräfte und Scherung. Beim Verlassen des Produktes aus der Gegenstrahlkammer war das Ergebnis eine heiße Nanoemulsion, die in den Vorphasenbehälter (3) über die Produktleitung 10 überführt wurde. Die Emulsion auf der Basis nanoskaliger Primärteilchen wurde warmgehalten, wobei nach 2 bis 3 Zyklen und Erhalt einer mittleren Partikelgröße von 150 bis 250 nm und einem Polydispersitätsindex von kleiner 0,25 die heiße Emulsion im Vorphasenbehälter (3) schockgekühlt wurde. Der Hochdruckhomogenisator ist mit einem Überdruckventil (11) (> 2000 bar), einem Bypassauslass (10) sowie einem Druckregler und einer Druckanzeige (14) ausgestattet, während der Vorphasenbehälter über eine Temperaturanzeige (15) verfügt. Die Herstellung der nanoskaligen Primärteilchen bzw. Lipidnanopartikel erfolgte batchweise und diskontinuierlich, wobei auch eine Semikontinuierliche Prozessführung durch kleine Änderungsmaßnahmen erfolgen kann.

Anschließend wurde das Zetapotential der Dispersionen bestimmt, dessen Betrag mehr als 30 mV sein sollte.

Die erhaltenen Dispersionen können entweder direkt weiterverarbeitet (konserviert) oder in geeignete sterile Behältnisse gefüllt und kühl gelagert werden. Ein Schema der für die Herstellung verwendeten Anlage ist in Fig. 1 wiedergegeben.

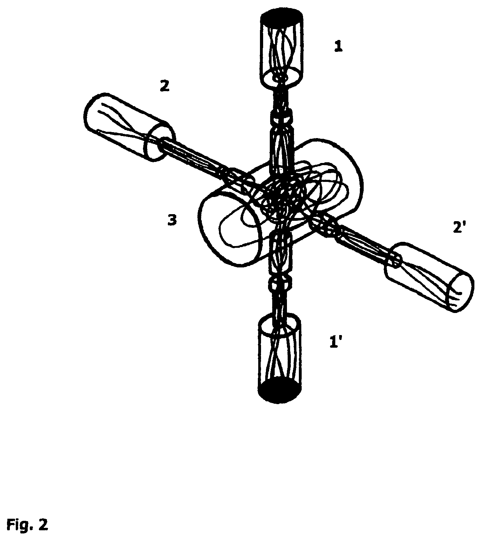

Eine schematische Darstellung des Homogenisationsprinzips des Gegenstrahlhomogenisators findet sich in Fig. 2, in der (1) und (1') Einlassdüsen, (2) und (2') Auslassdüsen sowie (3) die Stabilisierungskammer bezeichnet.

Für die vorstehend beschriebene Zusammensetzung 1 (Ceraparts^{®} Ω6) wurden bei einer Chargengröße von 2 Litern folgenden Eigenschaften bestimmt.

| **Sensorische Eiqenschaften** | |
|---|---|
| Geruch | produktspezifisch (leicht) |
| Farbe | weiß |
| Konsistenz | flüssig |
| **Analyse** | |
| pH-Wert | 3,5 bei 20 °C |
| **Partikelgrößenmessungen** | |

| | |
|---|---|
| ¹ = bestimmt mit Zetasizer Nano ZS | |

Bei der Teilchengrößenmessung zeigt es sich, dass ein Wert von 220 nm für die Teilchengröße erzielt werden konnte, der kleiner als die wünschenswerte Grenze von 300 nm ist. Ebenso wurde ein Polydispersitätsindex bestimmt, der kleiner als 0,25 ist. Der Polydispersitätsindex macht eine Aussage über die Einheitlichkeit der Teilchengröße. Ist der Wert klein, weist dies aus, dass die Produkte einheitlicher sind. Bei der Erfindung zeigt es sich, dass im Laufe der Alterung bezüglich dieser beiden angesprochenen Werte keine relevante Veränderung auftritt. Bei einer Vergleichsmessung veränderte sich die durch PSC bestimmte Partikelgröße innerhalb von 250 Tagen von 187 auf 202 nm, während die Polydispersität im gleichen Zeitraum von 0,14 auf 0,18 zunahm

## Patentansprüche

1. Wässrige Dispersion zur kosmetischen Applikation mit einem Gehalt an darin dispergierten, negativ oder positiv geladenen Primärteilchen einer Teilchengröße von 10 bis 1000 nm, wobei die Primärteilchen eine Lipidmatrix auf Basis von bei 20 °C festen Lipiden aufweisen und Wirkstoffe enthalten, **dadurch gekennzeichnet, dass** die Wirkstoffe amphiphil und in der Grenzfläche der Primärteilchen so verankert sind, dass deren lipophiler Anteil nach innen in die Primärteilchen und deren hydrophiler Anteil nach außen in die Dispersion weist, wobei die amphiphilen Wirkstoffe als Ceramide, Phytosterole, Lipoaminosäuren oder Lipoproteine vorliegen.

2. Wässrige Dispersion nach Anspruch 1, **dadurch gekennzeichnet, dass** sie 5 bis 70 Gew.-%, insbesondere 5 bis 45 Gew.-% nanoskalige Primärteilchen enthält.

3. Wässrige Dispersion nach Anspruch 1 oder 2, **dadurch gekennzeichnet, dass** das Zetapotential der Dispersion (bestimmt nach der Messmethode in Abhängigkeit vom pH-Wert mit dem Gerät Zetasizer Nano ZS, Malvern Instruments) zwischen 200 mV und 30 mV, entweder mit positivem oder negativem Vorzeichen, liegt.

4. Wässrige Dispersion nach Anspruch 3, **dadurch gekennzeichnet, dass** das Zetapotential zwischen 80 mV und 40 mV, entweder mit positivem oder negativem Vorzeichen, liegt.

5. Wässrige Dispersion nach mindestens einem der vorhergehenden Ansprüche, **dadurch gekennzeichnet, dass** die festen Lipide vorliegen in Form von Fettalkoholen, insbesondere Cetylalkohol, Fettsäureestern, insbesondere Partialfettsäureester, insbesondere in Form von Cetylpalmitat, Isopropylpermitat, Octylpalmitat, Fettsäuren, Steroiden, Terpenen, Saponinen, Wachsen, natürlich und/oder derivatisiert, insbesondere in Form von Bienenwachs und Canaubawachs, veresterte Proteine, insbesondere Ceramide (I und II), und/oder Phytosterole, dies sowohl natürlich als auch derivatisiert.

6. Wässrige Dispersion nach mindestens einem der vorhergehenden Ansprüche, **dadurch gekennzeichnet, dass** die amphiphilen Wirkstoffe in Form von Ceramiden als Aminopropandiolester, die Phytosterole als Phytosterole des Granatapfels oder der Acaibeere, die Lipoaminosäuren als Natriumcocoylalaninat oder Palmitoylglycin vorliegen.

7. Wässrige Dispersion nach Anspruch 6, **dadurch gekennzeichnet, dass** die amphiphilen Wirkstoffe in Form der Phytosterole als Phytosterol aus dem Granatapfel (Punica Granatum Sterolis), als Phytosterole aus der Acaibeere (Euterpe Oleracea Sterolis), als Leinsamenöl, als Palmöl, als Aminopropandiolester (synthetisches Ceramid), als Sonnenblumenöl, als PalmölAminopropanolester oder als Natriumcocoylalaninat (Lipoaminosäure) vorliegen.

8. Wässrige Dispersion nach mindestens einem der vorhergehenden Ansprüche, **dadurch gekennzeichnet, dass** die Menge an amphiphilen Wirkstoffen in den Primärteilchen 1 bis 60 Gew.-%, insbesondere 10 bis 45 Gew.-% beträgt.

9. Wässrige Dispersion nach mindestens einem der vorhergehenden Ansprüche, **dadurch gekennzeichnet, dass** die nanoskaligen Teilchen zusätzliche anwendungsspezifische Wirkstoffe für die Haut enthält, ausgewählt aus der Gruppe, umfassend Phytosterole aus dem Granatapfel, Phytosterole aus der Acaibeere, Lipoaminosäuren, Peptidestern, synthetische Ceramide, Ceramide I und II, lipophile/lipophilisierte Vitamine, Retinol, Tocopheron, Ubichinon, Ascorbyl-Palmitat oder Gemischen davon.

10. Wässrige Dispersion nach mindestens einem der vorhergehenden Ansprüche, **dadurch gekennzeichnet, dass** die nanoskaligen Primärteilchen zusätzliche Additive in Form von Antioxidationsmitteln, insbesondere Tocopherole, Ascorbyl-Palmitat, Retinol und Derivate hiervon, acylierte Polysaccharide, Feuchthaltemittel, wie Pentylenglycol oder Caprilylglycol, sowie Konservierungsmittel, insbesondere in Form von Phenoxyethanol oder Etylhexylglycerin, enthalten.

11. Wässrige Dispersion nach mindestens einem der vorhergehenden Ansprüche, **dadurch gekennzeichnet, dass** die nanoskaligen Primärteilchen angelagerte Stabilisatoren mit der Wirkung sterischer Hinderung aufweisen, insbesondere in Form von acylierten Polysacchariden und crosspolymerisierten, acylierten Acrylaten.

12. Wässrige Dispersion nach Anspruch 11, **dadurch gekennzeichnet, dass** die Stabilisatoren acylierte Polysaccharide, insbesondere auf Inulin-Basis, insbesondere Inulinlaurylcarbamat, oder auf Guar-Basis, insbesondere in Form von C₁₈-C₂₂-Hydroxyalkylhydroxypropyl-Guar, darstellen.

13. Verfahren zur Herstellung einer wässrigen Dispersion nach mindestens einem der vorhergehenden Ansprüche, **dadurch gekennzeichnet, dass** eine Präemulsion der Ausgangsmaterialen nach der PIT-Technologie (Phasen-Inversions-Temperatur/ Phasen-Volumen-Verhältnis) hergestellt, die Präemulsion einer Hochdruckhomogenisation unterzogen und anschließend die erhaltene wässrige Dispersion der nanoskaligen Primärteilchen abgekühlt wird.

14. Verfahren nach Anspruch 13, **dadurch gekennzeichnet, dass** eine Gegenstrahlhochdruckhomogenisation durchgeführt wird.

15. Verfahren nach Anspruch 13 oder 14, **dadurch gekennzeichnet, dass** bei der Hochdruckhomogenisation ein Druck von bis etwa 1300 bar, insbesondere von 800 bis 1200 bar, eingestellt wird.

16. Verfahren nach mindestens einem der Ansprüche 13 bis 15, **dadurch gekennzeichnet, dass** bei der Hochdruckhomogenisation ein Temperaturbereich von 30 bis 90°C insbesondere 40 bis 60°C eingestellt wird.

17. Verfahren nach mindestens einem der Ansprüche 13 bis 16, **dadurch gekennzeichnet, dass** das Kühlen mittels einer Schockkühlung durchgeführt wird.

18. Nicht-therapeutische Verwendung der wässrigen Dispersion nach mindestens einem der Ansprüche 1 bis 12 zur kosmetischen Applikation von Wirkstoffen, insbesondere auf die Haut.

19. Wässrige Dispersion nach mindestens einem der Ansprüche 1 bis 12 zur pharmazeutischen Applikation von Wirkstoffen, insbesondere auf die Haut.

20. Verwendung nach Anspruch 18 bzw. wässrige Dispersion nach Anspruch 19, **dadurch gekennzeichnet, dass** die Applikation auf die Barrieregeschädigte Haut erfolgt.

21. Verwendung oder Dispersion nach einem der Ansprüche 18 bis 20, **dadurch gekennzeichnet, dass** auf den nanoskaligen Teilchen ein immobilisierter weiterer Wirkstoff vorliegt.

## Claims

1. An aqueous dispersion for cosmetic application with a content of negatively or positively charged primary particles with a particle size of 10 to 1000 nm dispersed therein, wherein the primary particles have a lipid matrix based on lipids which are solid at 20ºC and include active substances, **characterised in that** the active substances are amphiphilic and are anchored in the boundary surface of the primary particles so that their lipophilic portion is directed inwardly into the primary particles and their hydrophilic portion is directed outwardly into the dispersion, wherein the amphiphilic active substances are present in the form of ceramides, phytosterols, lipoamino acids or lipoproteins.

2. An aqueous dispersion as claimed in Claim 1, **characterised in that** it contains 5 to 70 wt.%, particularly 5 to 45 wt.%, nanoscale primary particles.

3. An aqueous dispersion as claimed in Claim 1 or 2, **characterised in that** the zeta potential of the dispersion (determined by the measuring method in dependence on the pH value with the Zetasizer Nano ZS device, Malvern Instruments) is between 200 mV and 30 mV, with either a positive or negative algebraic sign.

4. An aqueous dispersion as claimed in Claim 3, **characterised in that** the zeta potential is between 80 mV and 40 mV, with either a positive or negative algebraic sign.

5. An aqueous dispersion as claimed in at least one of the preceding claims, **characterised in that** the solid lipids are present in the form of fatty alcohols, particularly cetyl alcohol, fatty acid esters, particularly partial fatty acid esters, particularly in the form of cetyl palmitate, isopropyl palmitate, octyl palmitate, fatty acids, steroids, terpenes, saponins, waxes, natural and/or derivatised, particularly in the form of beeswax and canauba wax, esterified proteins, particularly ceramides (I and II), and/or phytosterols, both natural and derivatised.

6. An aqueous dispersion as claimed in at least one of the preceding claims, **characterised in that** the amphiphilic active substances in the form of ceramides are present in the form of aminopropanediol esters, the phytosterols in the form of phytosterols of the pomegranate or the acai berry and the lipoamino acids in the form of sodium cocoyl alaninate or palmitoyl glycine.

7. An aqueous dispersion as claimed in Claim 6, **characterised in that** the amphiphilic active substances are present in the form of phytosterols in the form of phytosterol from the pomegranate (Punica Granatum Sterolis), in the form of phytosterols from the acai berry (Euterpe Oleracea Sterolis), in the form of linseed oil, in the form of palm oil, in the form of aminopropanediol ester (synthetic ceramide), in the form of sunflower oil, in the form of palm oil aminopropanol ester or in the form of sodium cocoyl alaninate (lipoamino acid).

8. An aqueous dispersion as claimed in at least one of the preceding claims, **characterised in that** the amount of amphiphilic active substances in the primary particles is 1 to 60 wt.%, particularly 10 to 45 wt.%.

9. An aqueous dispersion as claimed in at least one of the preceding claims, **characterised in that** the nanoscale particles contain additional application-specific active substances for the skin, selected from the group including phytosterols from pomegranates, phytosterols from acai berries, lipoamino acids, peptide esters, synthetic ceramides, ceramides I and II, lipophilic/lipophilised vitamins, retinol, tocopheron, ubichinon, ascorbyl palmitate or mixtures thereof.

10. An aqueous dispersion as claimed in at least one of the preceding claims, **characterised in that** the nanoscale primary particles contain additional additives in the form of antioxidants, particularly tocopherols, ascorbyl palmitate, retinol and derivatives thereof, acylated polysaccharides, wetting agents, such as pentylene glycol or caprylyl glycol, and preservatives, particularly in the form of phenoxyethanol or ethylhexylglycerine.

11. An aqueous dispersion as claimed in at least one of the preceding claims, **characterised in that** the nanoscale primary particles include added stabilisers with steric hindrance action, particularly in the form of acylated polysaccharides and cross-polymerised, acylated acrylates.

12. An aqueous dispersion as claimed in Claim 11, **characterised in that** the stabilisers represent acylated polysaccharides, particularly on an inulin basis, or on a guar basis, particularly in the form of C₁₈-C₂₂-hydroxyalkylhydroxypropyl-guar.

13. A method of producing an aqueous dispersion as claimed in at least one of the preceding claims, **characterised in that** a preemulsion of the starting materials is produced using the PIT technology (Phase-Inversion-Temperature/Phase-Volume-Relationship), the preemulsion is subjected to a high pressure homogenisation and subsequently the aqueous dispersion of the nanoscale primary particles which is obtained is cooled.

14. A method as claimed in Claim 13, **characterised in that** a countercurrent high pressure homogenisation is performed.

15. A method as claimed in Claim 13 or 14, **characterised in that** a pressure of up to about 1300 bar, particularly of 800 to 1200 bar, is set in the high pressure homogenisation.

16. A method as claimed in at least one of Claims 13 to 15, **characterised in that** a temperature range of 30 to 90ºC, particularly 40 to 60ºC, is set in the high pressure homogenisation.

17. A method as claimed in at least one of Claims 13 to 16, **characterised in that** the cooling is performed by means of shock cooling.

18. Non-therapeutic use of the aqueous dispersion as claimed in at least one of Claims 1 to 12 for cosmetic application of active substances, particularly to the skin.

19. An aqueous dispersion as claimed in at least one of Claims 1 to 12 for the pharmaceutical application of active substances, particularly to the skin.

20. Use as claimed in Claim 18 or aqueous dispersion as claimed in Claim 19, **characterised in that** the application is effected to barrier damaged skin.

21. Use or dispersion as claimed in one of Claims 18 to 20, **characterised in that** an immobilised further active substances is present on the nanoscale particles.

## Revendications

1. Dispersion aqueuse destinée à une application cosmétique, comprenant une fraction de particules primaires dispersées dans celle-ci et chargées négativement ou positivement, d'une taille de particules de 10 à 1000 nm, les particules primaires présentant une matrice lipidique à base de lipides solides à 20°C et contenant des substances actives, **caractérisée en ce que** les substances actives sont amphiphiles et sont ancrées au niveau de l'interface des particules primaires de sorte que leur fraction lipophile pointe vers l'intérieur des particules primaires et leur fraction hydrophile pointe vers l'extérieur de la dispersion, les substances actives amphiphiles se présentant sous la forme de céramides, de phytostérols, de lipoaminoacides ou de lipoprotéines.

2. Dispersion aqueuse selon la revendication 1, **caractérisée en ce qu'**elle contient 5 à 70 % en poids, notamment 5 à 45 % en poids de particules primaires nanoscalaires.

3. Dispersion aqueuse selon la revendication 1 ou 2, **caractérisée en ce que** le potentiel zêta de la dispersion (déterminé d'après la méthode de mesure en fonction du pH avec l'appareil Zetasizer Nano ZS, Malvern Instruments) se situe entre 200 mV et 30 mV, soit avec un signe positif soit un signe négatif.

4. Dispersion aqueuse selon la revendication 3, **caractérisée en ce que** le potentiel zêta se situe entre 80 mV et 40 mV, soit avec un signe positif soit un signe négatif.

5. Dispersion aqueuse selon au moins l'une des revendications précédentes, **caractérisée en ce que** les lipides solides se présentent sous la forme d'alcools gras, en particulier d'alcool cétylique, d'esters d'acide gras, en particulier d'ester d'acide gras partiel, notamment sous la forme de palmitate de cétyle, de permitate d'isopropyle, de palmitate d'octyle, d'acides gras, de stéroïdes, de terpènes, de saponines, de cires, naturelles et/ou dérivatisées, notamment sous la forme de cire d'abeilles et de cire de carnauba, de protéines estérifiées, en particulier de céramides (I et II) et/ou de phytostérols, ceux-ci étant bien entendu naturels ou bien dérivatisés.

6. Dispersion aqueuse selon au moins l'une des revendications précédentes, **caractérisée en ce que** les substances actives amphiphiles prenant la forme de céramides sont présentes en tant qu'esters d'aminopropanediol, les phytostérols en tant que phytostérols de la grenade ou des baies d'açaï, les lipoaminoacides en tant que cocoylalaninate de sodium ou de palmitoylglycine.

7. Dispersion aqueuse selon la revendication 6, **caractérisée en ce que** les substances actives amphiphiles prenant la forme de phytostérols sont présentes en tant que phytostérol provenant de la grenade (*Punica Granatum Sterolis*), en tant que phytostérols des baies d'açaï (*Euterpe Oleracea Sterolis*), en tant qu'huile de graines de lin, en tant qu'huile de palme, en tant qu'ester d'aminopropanediol (céramide synthétique), en tant qu'huile de tournesol, en tant qu'ester d'aminopropanediol-huile de palme ou en tant que cocoylalaninate de sodium (lipoaminoacide).

8. Dispersion aqueuse selon au moins l'une des revendications précédentes, **caractérisée en ce que** la quantité de substances amphiphiles dans les particules primaires est de 1 à 60 % en poids, en particulier de 10 à 45 % en poids.

9. Dispersion aqueuse selon au moins l'une des revendications précédentes, **caractérisée en ce que** les particules nanoscalaires contiennent des substances actives supplémentaires spécifiques à l'application pour la peau, choisies dans le groupe comprenant les phytostérols provenant de la grenade, les phytostérols provenant des baies d'açaï, les lipoaminoacides, les esters peptidiques, les céramides synthétiques, les céramides I et II, les vitamines lipophiles/lipophilisées, le rétinol, le tocophérol, l'ubiquinone, le palmitate d'ascorbyle ou des mélanges de ceux-ci.

10. Dispersion aqueuse selon au moins l'une des revendications précédentes, **caractérisée en ce que** les particules primaires nanoscalaires contiennent des additifs supplémentaires sous la forme d'agents antioxydants, notamment de tocophérols, de palmitate d'ascorbyle, de rétinol et de dérivés de ceux-ci, de polysaccharides acylés, d'agents hydratants, comme le pentylène glycol et le caprilyle glycol, ainsi que des conservateurs, notamment sous la forme de phénoxyéthanol ou de glycérine d'éthylhexyle.

11. Dispersion aqueuse selon au moins l'une des revendications précédentes, **caractérisée en ce que** les particules primaires nanoscalaires contiennent des stabilisants liés, ayant pour effet un encombrement stérique, notamment sous la forme de polysaccharides acylés et d'acrylates polymérisés de manière croisée et acylés.

12. Dispersion aqueuse selon la revendication 11, **caractérisée en ce que** les stabilisants sont des polysaccharides acylés, en particulier à base d'inuline, notamment de laurylcarbamate d'inuline ou à base de guar, en particulier sous la forme de (C₁₈-C₂₂hydroxy-alkyl)hydroxypropyle-guar.

13. Procédé de préparation d'une dispersion aqueuse selon au moins l'une des revendications précédentes, **caractérisé en ce qu'**une pré-émulsion des matières de départ est préparée d'après la technologie PIT (température d'inversion de phase / rapport phase - volume), ladite pré-émulsion étant soumise à une homogénéisation sous haute pression et la dispersion aqueuse de particules primaires nanoscalaires obtenue étant ensuite refroidie.

14. Procédé selon la revendication 13, **caractérisé en ce qu'**une homogénéisation sous haute pression à contre-jet est réalisée.

15. Procédé selon la revendication 13 ou 14, **caractérisé en ce que**, lors de l'homogénéisation sous haute pression, une pression allant jusqu'à environ 1300 bars, en particulier de 800 à 1200 bars, est ajustée.

16. Procédé selon au moins une des revendications 13 à 15, **caractérisé en ce que**, lors de l'homogénéisation sous haute pression, une plage de températures de 30 à 90°C, en particulier de 40 à 60°C, est ajustée.

17. Procédé selon au moins une des revendications 13 à 16, **caractérisé en ce que** le refroidissement est réalisé au moyen d'un refroidissement ultrarapide.

18. Utilisation non thérapeutique de la dispersion aqueuse selon au moins l'une des revendications 1 à 12 destinée à l'application cosmétique de substances actives, en particulier sur la peau.

19. Dispersion aqueuse selon au moins l'une des revendications 1 à 12 destinée à l'application pharmaceutique de substances actives, en particulier sur la peau.

20. Utilisation selon la revendication 18 ou dispersion aqueuse selon la revendication 19, **caractérisée en ce que** l'application s'effectue sur la peau dont la barrière a été détériorée.

21. Utilisation ou dispersion selon l'une des revendications 18 à 20, **caractérisée en ce qu'**une autre substance active immobilisée est présente sur les particules nanoscalaires.
